# EUROPEAN PATENT SPECIFICATION

(11) **EP 0 778 044 B1**
(45) Date of publication and mention of the grant of the patent: **01.09.2004**
(21) Application number: 96850204.7
(22) Date of filing: 03.12.1996
(51) Int. Cl.: A61M 25/09, A61N 1/05

(54) **Guidewire unit**
Führungsdrahteinheit
Fil de guidage

(30) Priority: 04.12.1995 SE 9504334
(43) Date of publication of application: 11.06.1997
(73) Proprietor: St. Jude Medical AB, 175 84 Järfälla (SE)
(72) Inventor: Boström, Mats, 172 31 Sundbyberg (SE); Lindegren, Ulf, 121 35 Enskededalen (SE)

(56) References cited:
- EP-A- 0 381 810
- WO-A-95/13111
- US-A- 3 757 768

## Description

### Field of the invention

The present invention relates to a stylet unit which can be inserted into a flexible component, such as a hollow electrode cable for a heart stimulator, a catheter or some other tubular instrument, with a narrow, longitudinal internal channel, to stiffen the flexible component and bend a distal end section of that component, said stylet unit constituting a double-stylet combination comprising a flexible, tubular stylet shell and an intemal stylet, displaceably arranged inside the shell's channel, with a pre-bent distal end section which can be set to a retracted position inside the stylet shell or to an exposed, projecting position outside the shell and whose radius of curvature is located on a first side of the internal stylet.

A channeled component of the aforementioned kind could e.g. be a tubular conductor used for stimulation in the human body. Such a channeled component could be devised to serve either as an implant or for removal from the body after a medical treatment has been performed.

### The prior art

A stylet unit of the present kind is especially suitable for stiffening and guiding a hollow electrode cable for a heart stimulator during the electrode cable's advancement into a human heart and for anchoring a contact electrode (electrode head) on the distal end of the cable in a cavity of the heart. The introduction of such an electrode cable into the heart is usually through a suitable vein, and the contact electrode can be anchored in the right ventricle or atrium. The temporarily introduced stylet unit inside the hollow electrode cable extends through the cable's central channel from the cable's proximal end (which is subsequently connected to the heart stimulator) to its distal end on which the contact electrode is located.

A stylet unit is especially suitable for anchoring a contact electrode in the heart's atrium, so an appropriate J shape can be imparted to the distal end section of the electrode cable, thereby facilitating introduction of the end section into the atrial auricle and anchoring of the contact electrode in the trabeculae of the atrial auricle. After the contact electrode has been anchored at the desired site in the heart, the stylet unit is completely removed from the heart.

EP 0 381 810 A1 refers to a catheter tip attitude controlling guide wire for use with a catheter adapted to pass over the guide wire. The guide wire comprises an elongated wire-like cylindrical shaft having a distal end, a proximal end and an intermediate portion with a plurality of bends along its length. The intermediate portion is provided with an enclosing shell-like helical coil means. However, this coil means, which may comprise interconnected coils, is merely arranged to protect the intermediate wire portion, and the ends of the coil means are secured, typically by solder, to the ends of the guide wire shaft. Consequently, the guide wire shaft is not axially displaceably arranged inside the channel of the coil means.

US A 3 757 768 refers to a spring guide-catheter comprising an inner wall portion defined by a helical spring, and an outer wall portion formed from a tube of plastic material. Inside the channel or lumen of the spring guide-catheter there is arranged a stylet wire for assisting in the manipulation and control of the spring guide-catheter. The stylet wire fits freely within said lumen and is freely movable axially with respect to the spring guide-catheter. However, this stylet wire is merely a one part single wire, and does not constitute any double stylet combination comprising a flexible, tubular stylet shell and an internal stylet wire displaceably arranged inside the shell's channel or lumen.

US A 5 170 787 describes (see FIG. 2 in the document) a stylet unit, comprising a double stylet combination with a flexible, tubular shell containing a moveable intemal stylet in the shell's central channel. At the proximal end of this known stylet unit, there is a maneuvering handle with which the shell and the internal stylet can be moved in relation to each other to retract the stylet's pre-curved distal end section into the surrounding shell's distal end section or to deploy the pre-curved distal end section of the stylet outside the opening of the shell's end section into the central channel of the distal end section of the surrounding electrode cable in order to impart the desired curved shape to the distal end section..

US A 4 136 703 shows another example of a stylet unit, devised as a double stylet combination, for an electrode cable. The stylet unit contains a pre-curved internal stylet in its distal end section.

However, the types of prior art stylet units described in the two last-mentioned documents, i.e. double stylet combinations, are incapable of insuring that the stylet unit - and accordingly its surrounding electrode cable - attains a desired, largely straight configuration or conformation when the intemal stylet's pre-bent distal end section is retracted into the corresponding distal end section of the stylet shell.

### Summary of the invention

The primary object of the present invention is to achieve a new type of stylet unit with which it is possible to attain a much straighter, i.e. with less lateral bending, and much better shape for the distal end section of the stylet shell, with a stylet unit devised as a double stylet combination, when the pre-bent distal end section of the displaceable internal stylet wire has been fully retracted into the shell's channel and is surrounded by the stylet shell's enclosing distal end section.

When the stylet's pre-bent distal end section has been exposed and deployed outside the opening of the stylet shell, it produces the desired bending of a corresponding section of the narrow, channel-equipped flexible component, which (as noted above) could be a hollow cable, a catheter or some other kind of elongate instrument posing some resistance to bending.

An additional object of the invention is to achieve a stylet unit whose internal stylet wire has a very pronounced J or fish hook shape at its distal end section when this section is fully deployed outside the distal end section of the stylet shell.

The design problems related to the aforementioned object are solved according to the present invention primarily when a stylet unit of the aforementioned kind has the distinguishing features set forth in the characterizing part of patent claim 1.

Preferred embodiments and refinements of the stylet unit according to the invention can also have the distinguishing features set forth in the dependent claims.

The main distinctive feature of the stylet unit according to the invention is that in the area located immediately before (viewed from the stylet unit's proximal end to its distal end) the pre-bent distal end section, the internal stylet is provided with a pre-shaped stylet section, comprising a pre-bent stylet section whose radius of curvature is located on an opposite side of the stylet in relation to the said first side of the stylet.

As a result of this combination of two diametrically opposed, pre-shaped curved stylet sections on the internal stylet wire, as proposed by the invention, the distal end section of the stylet unit's stylet shell displays a very stretched or flat S-shape when the internal stylet's double-bend end section has been completely retracted into the distal end section of a stylet shell.

With a stylet unit devised in this manner, the shape of the distal section of the stylet shell has an undulating shape so flat, when the "double-bend" end section of the stylet wire is fully retracted into shell, that the maximum deviation from the midline of the shell's otherwise straight section becomes far less than with a traditional stylet design only utilizing a distal end section with pre-bending on one side.

For the shape of the stylet shell to be as straight as possible, when the internal stylet is fully retracted into the shell, it would also be appropriate for the part of the internal stylet between the stylet's proximal end section and the stylet's pre-shaped stylet section, and the pre-bent distal end section, to have an essentially straight configuration in an unloaded state.

So it would be desirable for the internal stylet's pre-shaped stylet section and the stylet's pre-bent distal end section to lie on the same side of the longitudinal axis of said stylet part having an essentially straight configuration. Here, the centers of curvature for the stylet's pre-shaped stylet section and the stylet's pre-bent distal end section can then lie either on the same side of the said longitudinal axis or on opposite sides of this longitudinal axis. An optimally flat or longitudinally undulating S shape for the stylet shell, with the inner stylet retracted therein, is achieved when the stylet's s pre-shaped stylet section and the stylet's pre-bent distal end section are located in a common plane which then preferably also includes the said the stylet part having an essentially straight configuration.
The stylet wire's pre-bent distal end section is suitably designed and arranged (in relation to the straight part of the stylet, between the stylet's proximal end and the pre-shaped stylet section) so that this look-like, pre-bent distal end section is touched by the longitudinal axis of said straight stylet part.

### Description of drawings

The invention will now be exemplified, described and further explained, referring to the enclosed drawings which depict in an elucidatory manner, but not to scale, a currently preferred embodiment of a stylet unit and its internal stylet wire according to the invention: Thus:
FIG. 1 very schematically shows the relevant parts of a traditional stylet unit in which the internal stylet's pre-curved distal end section projects outside the stylet shell's opening;
FIG. 2 shows the stylet unit, depicted in FIG. 1, when the stylet's pre-curved distal end section has been retracted into the tubular stylet shell's distal end section;
FIG. 3 shows the internal stylet wire for a stylet unit, depicted in FIG. 4, according to the invention; and
FIG. 4 shows a stylet unit according to the invention when the stylet wire's pre-bent distal end section has been completely retracted into the stylet shell.

### The preferred embodiment

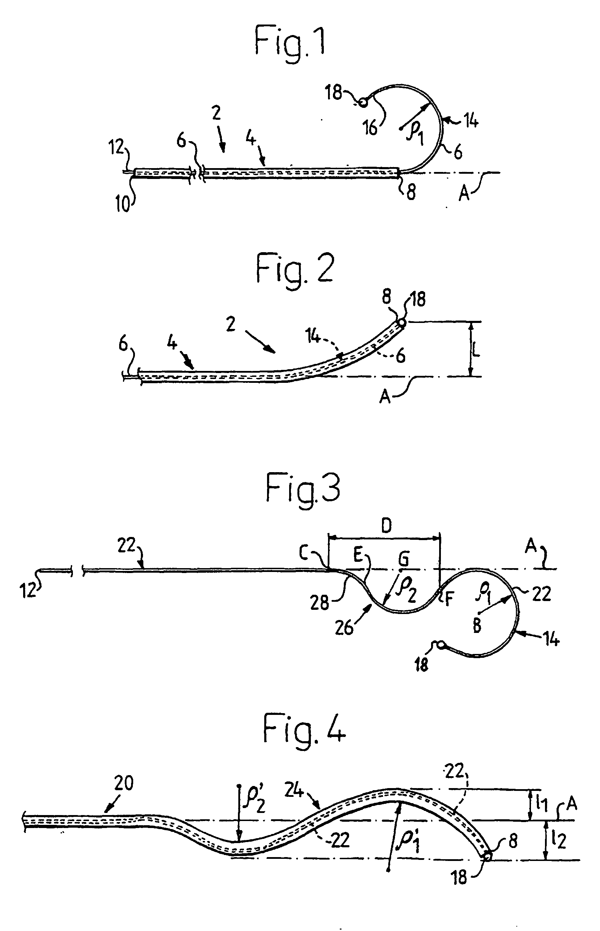

FIGS. 1-2 schematically show the distal end region of a known stylet unit 2, a double stylet combination consisting of a flexible, tubular stylet shell 4 with a freely moving stylet wire 6 inside the shell's 4 channel. The distal end of the stylet shell 4 is designated 8, the shell's proximal end is designated 10 and the stylet's 6 proximal end is designated 12. As FIG. 1 shows, the internal stylet 6 has a pre-bent distal end section 14 which, in this case, is mainly semicircular with a radius of curvature ρ₁ and a short, straight stylet end portion 16 with a end stop ball 18 which prevents the end section 14 from being unintentionally drawn too far into the stylet shell 4 and minimizes the risk of a surrounding electrode cable wall (not shown here) being penetrated by the stylet end portion 16. When the stylet unit 2 has been introduced into a longitudinally hollow electrode cable (not shown), the pre-bent distal end section 14 serves to impart a curved, J shape to the electrode cable's end section.

The stylet unit is shown in FIG. 2 as it might appear when the internal stylet wire's 6 pre-bent distal section 14 is completely retracted into the stylet shell's 4 end section, so the stop ball 18 presses against the opening on the distal end 8 of the tubular stylet shell. With the "single-bend" version of the stylet's distal end section 14, as shown in FIG. 1, a bend is produced in the stylet shell's 4 end section when the end section 14 is fully retracted. This means that the shell end 8 assumes a position corresponding to a lateral deviation L from the longitudinal axis A for the stylet unit's 2 straight section, shown to the left in FIGS. 1-2.

The relative movement required between the shell 4 and the stylet wire 6 to deploy or retract the end section 14 into/out of the stylet shell 4 is usually achieved with a maneuvering and holding implement device (not shown) arranged at the proximal end 10 of the shell 4. Here, the implement could be a holding device, for example, connected to the stylet shell 4, and a gripping device (handle), capable of moving the stylet in relation to the shell when the holding device is kept still. Alternately, movement between the internal stylet 6 and the surrounding shell 4 could be achieved by holding the stylet 6 still while moving the shell 4.

FIGS. 3-4 show an internal stylet wire and a stylet unit according to the invention.

The stylet unit, devised as a double stylet combination, according to the invention is generally designated 20, whereas the displaceably arranged internal stylet wire in the shell's channel is designated 22. This internal stylet wire 22 is shown in FIG. 3 in a completely unloaded state, i.e. before the stylet has been retracted into the stylet unit's 20 tubular stylet shell 24. As shown in FIG. 3, the internal stylet 22 has a pre-bent distal end section 14 of a known kind (cf. FIG. 1). This pre-bent semicircular end section 14 has a radius of curvature ρ₁ whose center of curvature B lies on the underside of the longitudinal axis A of the stylet wire's 22 straight stylet section between point C and the stylet's proximal end 12. The most distinguishing feature of the stylet wire according to the invention is that the stylet wire - over a region D - has a pre-shaped stylet section which comprises a pre-bent stylet section 26 with a radius of curvature ρ₂ and a semicircular transition section 28 between point E and point C. So the pre-bent stylet section 26 extends from point E to the flexion point F where the stylet's 22 pre-curved distal end section 14 begins.

As shown in FIG. 3, the stylet section's 26 radius of curvature is ρ₂ (from the center of curvature G) located on one side of the stylet wire 22, opposite the side where the pre-bent distal end section's 14 radius of curvature ρ₁ is located. Viewed along the stylet's 22 longitudinal axis from its proximal end 12, via points C, E and F, to the stop ball 18, the stylet section's 26 radius of curvature ρ₁ is on the stylet's left side whereas the end section's 14 radius of curvature ρ₁ is on the stylet's right side, seen on the FIGURE plane in FIG. 3.

As FIG. 3 also shows, the stylet's 22 pre-shaped stylet section and the stylet's pre-bent distal end section 14 are on the same side of the longitudinal axis A as the straight section of the intemal stylet 22 located between the proximal end 12 and the point C.

In the embodiment shown in FIG. 3, the radius of curvature's ρ₂ center of curvature G is on the longitudinal axis A, where as the radius of curvature's ρ₁ center of curvature B lies below said longitudinal axis A. However, the stylet's 22 pre-bent stylet section 26 could be devised so the centers of curvature G and B are on opposite sides of the longitudinal axis A.

Finally, FIG. 4 shows how the tubular stylet shell 24 in the unloaded state is bent laterally by the internal stylet's s 22 pre-bent stylet section 26 and by the pre-bent distal end section 14 when the two pre-bent parts of the stylet have been completely retracted into the tubular stylet shell 24, so the stop ball 18 presses against the opening of the shell's channel at the distal end 8 of the shell. In practice, retraction of the end section 14 and the stylet section 26 into the shell 24 is achieved by sliding the shell over both parts of the stylet. So the stylet shell 24 acquires a greatly flattened S shape with two regions, bent in opposite directions, on the shell's distal end section, viz. a concluding bent section with a radius of curvature ρ'₁ and a section before this, bent in the opposite direction ρ'₂. The stylet shell's 24 radius of curvature ρ'₁ is mainly caused by the pre-bent end section's 14 radius of curvature ρ₁ , whereas the shell's 24 radius of curvature ρ'₂ is mainly caused by the pre-bent stylet section's 26 radius of curvature ρ₂.

The wavy, double-bend distal end area of the stylet shell 24 will, as a result of the stylet's 22 special conformation, display maximum lateral bending l₁ and l₂, seen from the stylet unit's longitudinal axis A. But both the deviation l₁ and the deviation l₂ will be much less than the lateral deviation L (see FIG. 2) obtained with the use of a stylet shell 4 of the same kind as the stylet shell 24 used with the conventional type of internal stylet 6, according to FIG. 1. Even if the sum of the deviations l₁ and l₂ were as large as the lateral deviation L, according to FIG. 2, a maximum deviation from the longitudinal axis A, which is much less than the deviation L obtained with the known technic according to FIGS. 1-2. The deviation-reducing effect attained according to the invention is because the lateral deviations l₁ and l₂ are located on opposite sides of the center of longitudinal axis A.

As the above shows, the stylet unit according to the invention is especially suitable for achieving a stiffening of an electrode cable (or lead) during the cable's advancement through a vein to a patient's heart and in concluding introduction a bending of a distal end section of the cable. During the electrode cable's advancement through a vein, it would obviously be advantageous for the electrode cable to be as straight as possible. This means, in turn, that the employed stylet unit must be as straight as possible during advancement, since it is the stylet unit which gives the electrode cable its stiffness. So a stylet unit according to FIG. 4 would greatly simplify a physician's introduction of an electrode cable into a patient's heart compared to the use of a conventional stylet unit (according to FIG. 2) which imparts much greater lateral bending than the double stylet type of curved stylet unit, shown in FIG. 4, according to the present invention.

## Claims

1. A stylet unit (20) which can be inserted into a flexible component, such as a hollow electrode cable, catheter or some other tubular instrument, with a narrow, longitudinal, internal channel, to stiffen the flexible component and bend a distal end section of that component, said stylet unit constituting of a double stylet combination comprising a flexible, tubular stylet shell (24) and an internal stylet (22), displaceably arranged inside the shell's channel, with a pre-bent distal end section (14) which can be set to a retracted position inside the shell or to a position projecting outside the shell and whose radius of curvature (ρ₁) is located on a first side of the internal stylet (22), **characterized in that** the intemal stylet (22) is provided with a pre-shaped stylet section, in an area (D) located before the pre-bent distal end section (14), comprising a pre-bent stylet section (26) whose radius of curvature (ρ₂) is located on an opposite second side of the stylet in relation to the said first side of the stylet (22).

2. A stylet unit according to claim 1, **characterized in that** the part of the internal stylet (22) between a proximal end section of the stylet and the stylet's pre-shaped stylet section and the connected pre-bent distal end section (14) has an essentially straight configuration in an unloaded state.

3. A stylet unit according to claim 2, **characterized in that** the stylet's (22) pre-shaped stylet section and the stylet's s pre-bent distal end section (14) are on the same side of the longitudinal axis (A) of said stylet part having an essentially straight configuration.

4. A stylet unit according to claim 3, **characterized in that** the centers of curvature (G, B) for the stylet's s pre-shaped stylet section and the stylet's pre-bent end section (14) are on the same side of the said longitudinal axis (A).

5. A stylet unit according to claim 3, **characterized in that** the centers of curvature (G, B) for the stylet's pre-shaped stylet section and the stylet's pre-bent end section (14) are on different sides of the said longitudinal axis (A).

6. A stylet unit according to any of the preceding claims, **characterized in that** the radii of curvature (ρ₂, ρ₁) for the stylet's (22) pre-shaped stylet section and the stylet's pre-bent end section (14) are at least essentially constant for the respective section.

7. A stylet unit according to any of claims 2-6, **characterized in that** the stylet's (22) pre-shaped stylet section and the stylet's pre-bent end section (14) are located in a common plane preferably also including said stylet part having an essentially straight configuration.

8. A stylet unit according to any of claims 3-7, **characterized in that** the stylet's (22) pre-bent distal end section (14) is designed and arranged so that it is touched by the longitudinal axis A of said stylet part having an essentially straight configuration.

## Patentansprüche

1. Mandrineinheit (20), die in eine flexible Komponente, wie ein hohles Elektrodenkabel, einen Katheder oder ein anderes rohrförmiges Instrument mit einem engen, longitudinalen inneren Kanal einsetzbar ist, um die flexible Komponente zu versteifen und einen distalen Endabschnitt der Komponente zu biegen, wobei die genannte Mandrineinheit eine doppelte Mandrinkombination mit einer flexiblen rohrförmige Mandrinhülle (24) und einem inneren Mandrin (22) umfasst, der in dem Kanal der Hülle verschiebbar angeordnet ist, mit einem vorgebogenen distalen Endabschnitt (14), der in eine zurückgezogene Position innerhalb der Hülle oder in eine aus der Hülle vorstehende Position gebracht werden kann und dessen Kurvenradius (ρ₁) an einer ersten Seite des inneren Mandrins (22) gelegen ist, **dadurch gekennzeichnet, dass** der innere Mandrin (22) mit einem vorgeformten Mandrinabschnitt in einem vor dem vorgebogenen distalen Endabschnitt (14) gelegenen Bereich (D) versehen ist, einen vorgebogenen Mandrinabschnitt (26) enthält, dessen Krümmungsradius (ρ₂) an einer bezüglich der genannten ersten Seite des Mandrins (22) entgegengesetzten zweiten Seite des Mandrins gelegen ist.

2. Mandrineinheit nach Anspruch 1, **dadurch gekennzeichnet, dass** der Teil des inneren Mandrins (22) zwischen einem proximalen Endabschnitt des Mandrins und dem vorgeformten Mandrinabschnitt des Mandrins sowie dem verbundenen vorgebogenen distalen Endabschnitt (14) im unbelasteten Zustand eine im wesentlichen gerade Konfiguration aufweist.

3. Mandrineinheit nach Anspruch 2, **dadurch gekennzeichnet, dass** der vorgeformte Mandrinabschnitt des Mandrins (22) und der vorgebogene distale Endabschnitt (14) des Mandrins an der gleichen Seite der longitudinalen Achse (A) des genannten Mandrinteils mit einer im wesentlichen geraden Konfiguration sind.

4. Mandrineinheit nach Anspruch 3, **dadurch gekennzeichnet, dass** die Krümmungsmittelpunkte (G, B) für den vorgeformten Mandrinabschnitt des Mandrins und den vorgebogenen Endabschnitt (14) des Mandrins auf der gleichen Seite der genannten Längsachse (A) liegen.

5. Mandrineinheit nach Anspruch 3, **dadurch gekennzeichnet, dass** die Krümmungsmittelpunkte (G, B) für den vorgeformten Mandrinabschnitt des Mandrins und den vorgebogenen Endabschnitt (14) des Mandrins an verschiedenen Seiten der genannten Längsachse (A) liegen.

6. Mandrineinheit nach einem der vorhergehenden Ansprüche, **dadurch gekennzeichnet, dass** die Krümmungsradien (ρ₂,ρ₁) für den vorgeformten Mandrinabschnitt des Mandrins (22) und den vorgebogenen Endabschnitt (14) des Mandrins für den betreffenden Abschnitt wenigstens im wesentlichen konstant sind.

7. Mandrineinheit nach einem der Ansprüche 2 bis 6, **dadurch gekennzeichnet, dass** der vorgeformte Mandrinabschnitt des Mandrins (22) und der vorgebogene Endabschnitt (14) des Mandrins in einer gemeinsamen Ebene, die vorzugsweise auch den Mandrinteil mit im wesentlicher gerader Konfiguration enthält, gelegen sind.

8. Mandrineinheit nach einem der Ansprüche 3 bis 7, **dadurch gekennzeichnet, dass** der vorgebogene Endabschnitt (14) des Mandrins so ausgebildet und angeordnet ist, dass er durch die Längsachse (A) des genannten Mandrinteils mit einer im wesentlichen geraden Konfiguration tangiert wird.

## Revendications

1. Unité (20) à stylet, qui peut être insérée dans un élément souple, tel qu'un câble tubulaire d'électrode, un cathéter ou quelque autre instrument tubulaire, ayant un canal intérieur longitudinal étroit pour raidir l'élément souple et courber une section d'extrémité distale de cet élément, l'unité à stylet constituant une combinaison double à stylet comprenant une gaine (24) de stylet souple et tubulaire et un stylet (24) intérieur monté mobile dans le canal de la gaine, ayant une section (14) d'extrémité distale précourbée qui peut être mise en une position rétractée à l'intérieur de la gaine ou en une position faisant saillie à l'extérieur de la gaine et dont le rayon de courbure (ρ₁) est placé sur un premier côté du stylet (22) intérieur, **caractérisée en ce que** le stylet (22) intérieur est muni d'une section de stylet préformée dans une zone (D) placée avant la section (14) d'extrémité distale précourbée, comprenant une section (26) de stylet précourbée, dont le rayon de courbure (ρ₂) est placé sur un deuxième côté du stylet opposé au premier côté du stylet (22).

2. Unité à stylet suivant la revendication 1, **caractérisée en ce que** la partie du stylet (22) intérieur entre une section d'extrémité proximale du stylet et la section préformée et la section (14) d'extrémité distale précourbée et reliée du stylet a une configuration sensiblement rectiligne dans un état non chargé.

3. Unité à stylet suivant la revendication 2, **caractérisée en ce que** la section préformée du stylet (22) et la section (14) d'extrémité distale précourbée du stylet sont du même côté de l'axe (A) longitudinal de la partie du stylet ayant une configuration essentiellement rectiligne.

4. Unité à stylet suivant la revendication 3, **caractérisée en ce que** les centres de courbure (G, B) de la section préformée du stylet et de la section (14) d'extrémité précourbée du stylet sont du même côté de l'axe (A) longitudinal.

5. Unité à stylet suivant la revendication 3, **caractérisée en ce que** les centres de courbure (G, B) de la section préformée du stylet et de la section (14) d'extrémité précourbée du stylet sont sur des côtés différents de l'axe (A) longitudinal.

6. Unité à stylet suivant l'une quelconque des revendications précédentes, **caractérisée en ce que** les rayons de courbure (ρ₂, ρ₁) de la section préformée du stylet (22) et de la section (14) d'extrémité précourbée du stylet sont au moins sensiblement constants pour la section respective.

7. Unité à stylet suivant l'une quelconque des revendications 2 à 6, **caractérisée en ce que** la section préformée du stylet (22) et la section (14) d'extrémité précourbée du stylet sont dans un plan commun passant de préférence aussi par la partie du stylet ayant une configuration essentiellement rectiligne.

8. Unité à stylet suivant l'une quelconque des revendications 3 à 7, **caractérisée en ce que** la section (14) d'extrémité distale précourbée du stylet (22) est conçue et disposée de manière à être touchée par l'axe (A) longitudinal de la partie du stylet ayant une configuration essentiellement rectiligne.
